# EUROPEAN PATENT SPECIFICATION

(11) **EP 2 401 952 B1**
(45) Date of publication and mention of the grant of the patent: **28.12.2016**
(21) Application number: 10846623.6
(22) Date of filing: 29.11.2010
(51) Int. Cl.: A61B 1/00, A61B 17/28

(54) **ENDOSCOPE APPARATUS**
ENDOSKOPVORRICHTUNG
APPAREIL D'ENDOSCOPIE

(30) Priority: 26.02.2010 JP 2010042571
(43) Date of publication of application: 04.01.2012
(73) Proprietor: Olympus Corporation, Tokyo 192-8507 (JP)
(72) Inventor: TAKAHASHI, Kazuhiko, Hachioji-shi, Tokyo 192-8512 (JP)
(74) Representative: Schicker, Silvia
(86) International application number: PCT/JP2010/071241
(87) International publication number: WO 2011/104960

(56) References cited:
- WO-A1-2006/129726
- WO-A1-2007/096950
- DE-A1-102007 008 272
- JP-A- 8 224 244
- JP-A- 2007 215 786
- JP-A- 2007 532 262
- JP-A- 2009 142 513
- JP-A- 2010 035 668
- JP-A- 2011 000 326
- US-A1- 2008 021 274
- US-A1- 2009 182 194

## Description

### Technical Field

The present invention relates to an endoscope device which includes an endoscope, and a treatment instrument such as a manipulator or a pair of forceps to be inserted through a treatment instrument insertion passage provided in the endoscope or in a tubular member separate from the endoscope.

### Background Art

In a generally used endoscope device, an endoscope is inserted into a body cavity, and a treatment instrument such as a manipulator or a pair of forceps is projected from the distal end of the endoscope or a tubular member via a treatment instrument insertion passage in the endoscope or in the tubular member separate from the endoscope. In the endoscope device, the treatment instrument allows a treatment in the body cavity to be carried out under endoscopic observation.

As a treatment instrument to be inserted through a treatment instrument insertion passage in an endoscope or in a tubular member, Patent Literature 1 has disclosed a high-frequency treatment instrument which grips and operates on an affected part by a high-frequency electrode. This high-frequency surgical instrument includes an insertion portion to be inserted into a body cavity, and an operation portion provided in a proximal direction side to the insertion portion. The insertion portion includes a distal treatment portion provided with the high-frequency electrode, and a treatment instrument body which is provided in the proximal direction side to the distal treatment portion and which is extended in a longitudinal direction. In order to rotate the distal treatment portion, rotation torque produced by rotational operation in the operation portion is transmitted to the distal treatment portion via a conductive wire which is a rotational operation transmission member to be inserted through the treatment instrument body, and the distal treatment portion is rotated around the axis relative to the treatment instrument body.

There is also a treatment instrument in which a motor is provided between a distal treatment portion and a treatment instrument body. In this treatment instrument, a motor is driven to rotate the distal treatment portion around the axis relative to the treatment instrument body. That is, the treatment instrument includes a rotary joint portion that allows the distal treatment portion to be rotated relative to the treatment instrument body by the motor.

Moreover, there is also a treatment instrument in which a bevel gear is provided between a distal treatment portion and a treatment instrument body, and the bevel gear is rotated to rotate the distal treatment portion. In this treatment instrument, a rotational operation transmission member such as a wire to be inserted through the treatment instrument body is connected to the bevel gear. When the wire is pulled and pushed out, the bevel gear is rotated, and the distal treatment portion is rotated around the axis relative to a flexible tube. That is, the treatment instrument includes a rotary joint portion that allows the distal treatment portion to be rotated relative to the treatment instrument body by the bevel gear.

Document US 2009/182194, on which document is based the preamble of claim 1, discloses a structure to prevent a rotation of a treatment instrument with respect to an endoscope which makes use of a pull wire or an inflating bladder.

Document US 2008/0021274 discloses an outer locking device and a medical device which is inserted through the outer locking device. The outer locking device includes ribs, and the medical device includes locking elements configured to engage the ribs. When the locking elements engage the ribs, a rotation of medical device with respect to the outer locking device is prevented.

### Citation List

### Patent Literature

Patent Literature 1: Jpn. Pat. Appln. KOKAI Publication No. 2009-142513

### Summary of Invention

### Technical Problem

In the high-frequency treatment instrument disclosed in Patent Literature 1, the conductive wire which transmits the rotation torque produced in the operation portion is extended in the longitudinal direction, so that the rotation torque at the proximal end side of the conductive wire may not be properly transmitted to the distal treatment portion. In this case, the rotation following performance of the distal treatment portion decreases, so that the degree of rotation in the operation portion may vary from rotational force in the distal treatment portion, or the rotation of the distal treatment portion may be uneven.

In the treatment instrument in which the rotary joint portion which includes, for example, the motor or the bevel gear provided between the distal treatment portion and the treatment instrument body is provided, there is no problem of the decreased rotation following performance of the distal treatment portion. However, in this treatment instrument, when, for example, a gripping portion provided in the distal treatment portion grips a tissue or the like, force is applied to the gripping portion provided in the distal direction side to the rotary joint portion. When the distal treatment portion is rotated in this condition, the distal treatment portion in the distal direction side to the rotary joint portion may not be rotated, and a portion in the proximal direction side to the rotary joint portion may be rotated in a direction opposite to the rotational direction of the rotational operation. That is, when force is applied to the distal treatment portion, the rotational operation may not be properly transmitted to the distal treatment portion.

The present invention has been made in consideration of the above problems, and is intended to provide an endoscope device in which rotational operation can be properly transmitted to the distal treatment portion, even if force is applied to the distal treatment portion.

### Solution to Problem

To achieve the above target, according to one aspect of the present invention, an endoscope device according to claim 1 is provided.

According to the present invention, it is able to provide an endoscope device in which rotational operation can be properly transmitted to the distal treatment portion, even if force is applied to the distal treatment portion.

### Brief description of Drawings

FIG. 1 is a schematic diagram showing an endoscope device according to a first embodiment helpful for understanding the present invention;
FIG. 2 is a block diagram showing the endoscope device according to the first embodiment;
FIG. 3 is a perspective view showing the configuration of the distal direction side part of the endoscope device according to the first embodiment;
FIG. 4 is a sectional view taken along line IV-IV of FIG. 3;
FIG. 5 is a plan view showing the configuration of the distal direction side part of a treatment instrument of an endoscope device according to an embodiment of the present invention;
FIG. 6 is a sectional view taken along line VI-VI of FIG. 5;
FIG. 7 is a sectional view showing the internal configuration of a treatment instrument insertion passage in an endoscope device according to a modification of the first and second embodiments of the present invention;
FIG. 8 is a block diagram showing the configuration of a treatment instrument of an endoscope device according to an embodiment helpful for understanding the present invention; and
FIG. 9 is a schematic diagram showing the configuration of the distal direction side part of an endoscope device according to a modification of the present invention in which a treatment instrument insertion passage is provided in a tubular member separate from an endoscope.

### Description of Embodiments

### (First Embodiment)

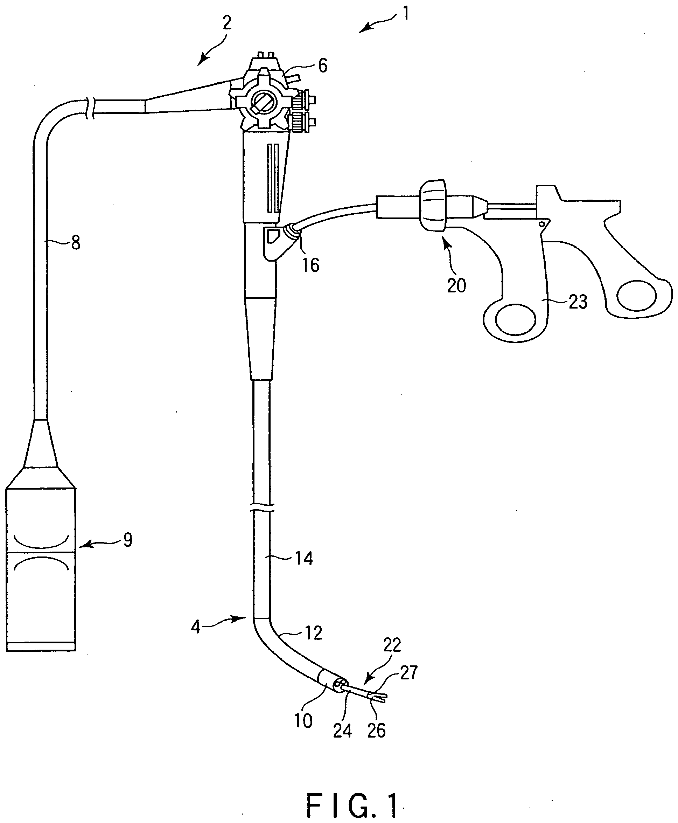

A first embodiment helpful for understanding the present invention is described with reference to FIG. 1 to FIG. 4.

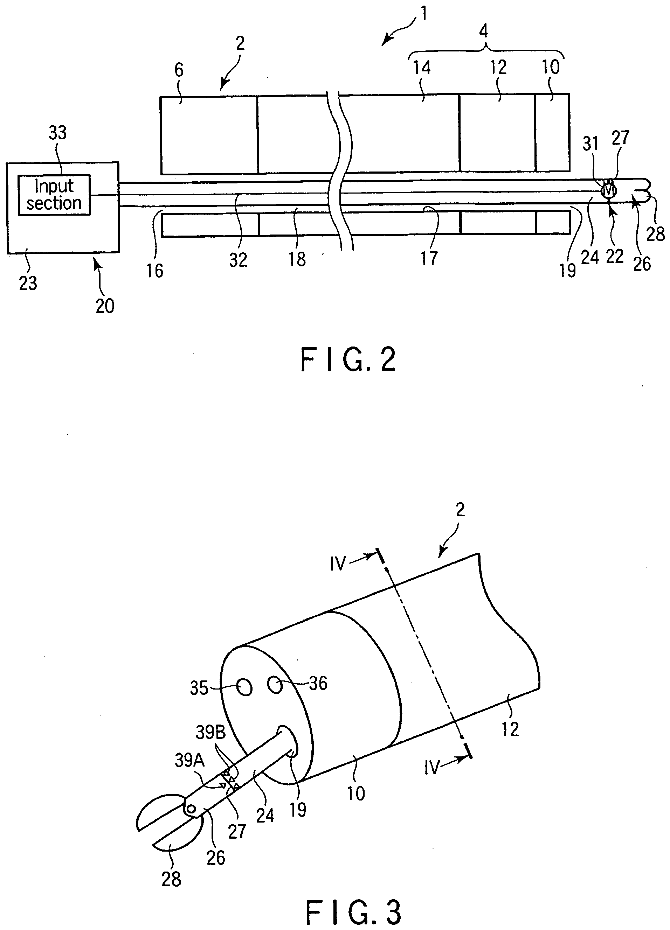

FIG. 1 and FIG. 2 are diagrams showing an endoscope device 1. As shown in FIG. 1, the endoscope device 1 includes an endoscope 2 configured to image a subject such as an affected part. The endoscope 2 includes an endoscope insertion portion 4 to be inserted into a body cavity, and an endoscope operation portion 6 provided in a proximal direction side to the endoscope insertion portion 4. One end of a universal cord 8 is connected to the endoscope operation portion 6. The other end of the universal cord 8 is connected to, for example, an image observation unit and an illumination power supply unit (none of which are shown) via a scope connector 9.

The endoscope insertion portion 4 includes a rigid distal hard portion 10, an endoscope curving portion 12 which is provided in the proximal direction side to distal hard portion 10 and which is configured to be curved, and a long and flexible endoscope flexible tube 14 provided in the proximal direction side to endoscope curving portion 12.

As shown in FIG. 2, a treatment instrument insertion hole 16 is provided in the endoscope operation portion 6 of the endoscope 2. An inner peripheral surface 17 extends from the treatment instrument insertion hole 16 to the distal hard portion 10 through the endoscope flexible tube 14 and the endoscope curving portion 12. A treatment instrument insertion passage 18 is defined by the inner peripheral surface 17. A treatment instrument hole 19 which is the distal end of the treatment instrument insertion passage 18 is provided in the distal face of the distal hard portion 10.

A treatment instrument 20 is configured to be inserted through the treatment instrument insertion passage 18 of the endoscope 2 movably back and forth in the longitudinal direction. The treatment instrument 20 includes a treatment instrument insertion portion 22 to be inserted into a body cavity, and a treatment instrument operation portion 23 provided in the proximal direction side to the treatment instrument insertion portion 22. The treatment instrument insertion portion 22 includes a flexible treatment instrument body 24 extended in the longitudinal direction, and a distal treatment portion 26 which is provided in a distal direction side to the treatment instrument body 24 and which is configured to allow a treatment to be carried out. A rotary joint portion 27 is provided between the treatment instrument body 24 and the distal treatment portion 26. When the rotary joint portion 27 is actuated, the distal treatment portion 26 is rotated around the axis relative to the treatment instrument body 24. The distal treatment portion 26 is provided with a gripping portion 28 configured to grip a tissue or the like.

As shown in FIG. 2, the rotary joint portion 27 is provided with a motor 31. The motor 31 is connected, via an electric wiring line 32, to an input section 33 provided in the treatment instrument operation portion 23. In response to rotational operation in the input section 33, the motor 31 is driven, and the rotary joint portion 27 is actuated. As a result of the actuation of the rotary joint portion 27, the distal treatment portion 26 is rotated around the axis relative to the treatment instrument body 24. A gripping operation transmission member such as a wire (not shown) is connected to the input section 33. The gripping operation in the input section 33 is transmitted to the gripping portion 28 via the gripping operation transmission member. As a result, the gripping portion 28 performs gripping action of gripping a tissue or the like.

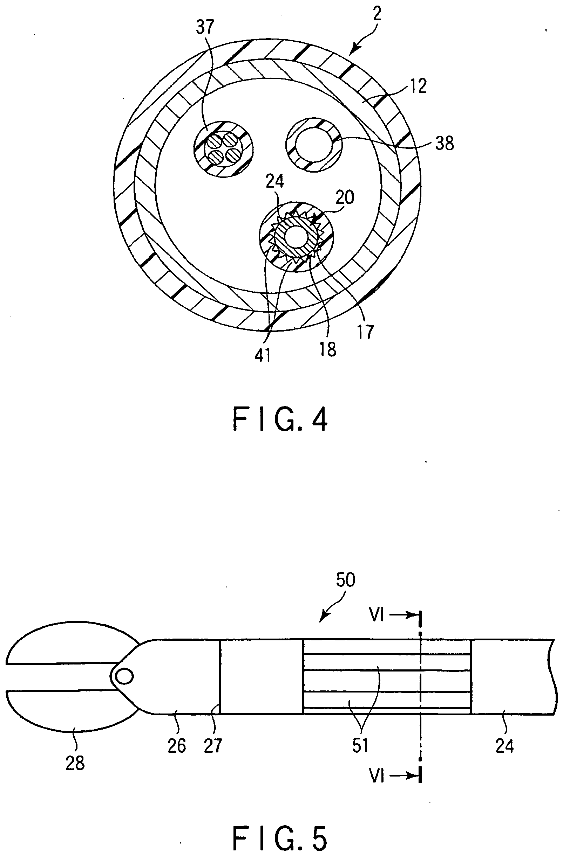

FIG. 3 is a diagram showing the configuration of the distal direction side part of the endoscope device 1. FIG. 4 is a sectional view taken along line IV-IV of FIG. 3. As shown in FIG. 3, an observation window 35 and an illumination window 36 are provided in the distal face of the distal hard portion 10 of the endoscope 2. An image pickup element (not shown) provided in the distal hard portion 10 is configured to image the subject via the observation window 35. An imaging cable 37 (see FIG. 4) is connected to the image pickup element. The imaging cable 37 is connected to an image observation unit (not shown) through the endoscope insertion portion 4, the endoscope operation portion 6, and the universal cord 8. A subject image taken by the image pickup element is output to the image observation unit via the imaging cable 37. A light guide 38 (see FIG. 4) is optically connected to the illumination window 36. The light guide 38 is optically connected to an illumination power supply unit (not shown) through the endoscope insertion portion 4, the endoscope operation portion 6, and the universal cord 8. Light exiting from the illumination power supply unit is guided by the light guide 38, and applied to the subject via the illumination window 36.

When the treatment instrument 20 is inserted through the treatment instrument insertion passage 18, the treatment instrument 20 is projected toward the distal direction from the treatment instrument hole 19 provided in the distal face of the distal hard portion 10. A first index 39A is provided in the outer peripheral surface of the distal treatment portion 26, and second indexes 39B are provided in the outer peripheral surface of the treatment instrument body 24. Thus, an operator checks the positional relation between the first index 39A and the second indexes 39B from the image taken by the image pickup element of the endoscope 2. From the positional relation between the first index 39A and the second indexes 39B, it is possible to recognize the neutral position (initial position) of the distal treatment portion 26, and the degree of rotation of the distal treatment portion 26 around the axis relative to the instrument body 24 from the neutral position. Therefore, whether the rotational operation in the input section 33 is properly transmitted can be recognized.

As shown in FIG. 4, in the distal direction side part of the inner peripheral surface 17 of the endoscope 2, projections 41 projecting toward the inner peripheral side are provided side by side in the circumferential direction of the treatment instrument insertion passage 18. The projections 41 are provided in a state that the projections 41 have a predetermined length in the longitudinal direction. When the treatment instrument 20 is inserted through the treatment instrument insertion passage 18, each of the projections 41 is in contact with the outer peripheral surface of the treatment instrument body 24. In such a configuration, when the treatment instrument body 24 is rotated around the axis relative to the endoscope 2, frictional force is applied in a direction opposite to the rotational direction of the treatment instrument body 24 at a contact portion between the projections 41 and the outer peripheral surface in the distal direction side part of the treatment instrument body 24. That is, the projections 41 serve as a frictional force application portion which is configured to apply the frictional force to the contact portion between the projections 41 (distal direction side part of the inner peripheral surface 17) and the outer peripheral surface in the distal direction side part of the treatment instrument body 24. The frictional force applied by the projections 41 regulates the rotation of the distal direction side part of the treatment instrument body 24 around the axis. The back-and-forth motion of the treatment instrument 20 relative to the endoscope 2 in the longitudinal direction is not regulated by the projections 41.

Now, the function of the endoscope device 1 according to the present embodiment is described. In the endoscope device 1, the treatment instrument 20 is inserted through the treatment instrument insertion passage 18 of the endoscope 2 to carry out a treatment. In the treatment using the endoscope device 1, a tissue or the like may be gripped by the gripping portion 28 of the distal treatment portion 26. During the gripping action performed by the gripping portion 28, force is applied to the gripping portion 28 provided in the distal direction side to the rotary joint portion 27. If the rotary joint portion 27 is actuated in this condition to rotate the distal treatment portion 26, the distal direction side part of the treatment instrument body 24 provided in the proximal direction side to the rotary joint portion 27 rotates in a direction opposite to the rotational direction of the rotational operation.

In the endoscope device 1, each of the projections 41 is in contact with the outer peripheral surface of the treatment instrument body 24 when the treatment instrument 20 is inserted through the treatment instrument insertion passage 18. Therefore, when the treatment instrument body 24 is rotated around the axis relative to the endoscope 2, frictional force is applied in a direction opposite to the rotational direction of the treatment instrument body 24 at the contact portion between the projections 41 (distal direction side part of the inner peripheral surface 17) and the outer peripheral surface in the distal direction side part of the treatment instrument body 24. The frictional force applied by the projections 41 regulates the rotation of the distal direction side part of the treatment instrument body 24 in a direction opposite to the rotational direction of the rotational operation. The around-the-axis rotation of the distal direction side part of the treatment instrument body 24 provided in the proximal direction side to the rotary joint portion 27 is regulated. As a result, even when the gripping portion 28 is gripping a tissue or the like (force is being applied to the distal treatment portion 26), the rotational operation is properly transmitted to the distal treatment portion 26 provided in the distal direction side to the rotary joint portion 27. Thus, the distal treatment portion 26 properly rotates around the axis relative to the treatment instrument body 24.

Therefore, the endoscope device 1 having the configuration described above has the following advantages. That is, in the endoscope device 1 according to the present embodiment, when the treatment instrument 20 is inserted through the treatment instrument insertion passage 18, each of the projections 41 is in contact with the outer peripheral surface of the treatment instrument body 24. Therefore, when the treatment instrument body 24 is rotated around the axis relative to the endoscope 2, frictional force is applied in a direction opposite to the rotational direction of the treatment instrument body 24 at the contact portion between the projections 41 (distal direction side part of the inner peripheral surface 17) and the outer peripheral surface in the distal direction side part of the treatment instrument body 24. The frictional force applied by the projections 41 regulates the rotation of the distal direction side part of the treatment instrument body 24 in a direction opposite to the rotational direction of the rotational operation when the distal treatment portion 26 is rotated in a state that the gripping portion 28 is gripping a tissue or the like (force is being applied to the distal treatment portion 26). The around-the-axis rotation of the distal direction side part of the treatment instrument body 24 provided in the proximal direction side to the rotary joint portion 27 is regulated. As a result, even when the force is being applied to the distal treatment portion 26, the rotational operation can be properly transmitted to the distal treatment portion 26 provided in the distal direction side to the rotary joint portion 27.

### (Second Embodiment)

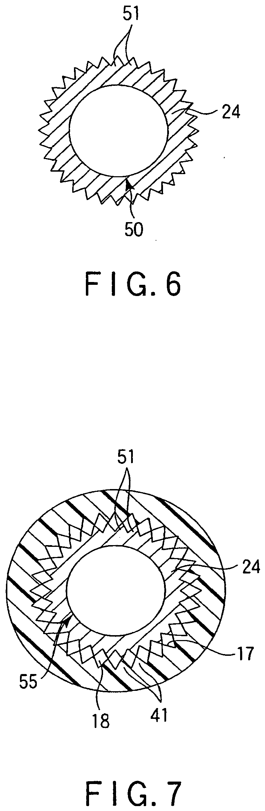

Now, a second embodiment is described with reference to FIG. 5 and FIG. 6. It should be noted that components having the same function as those in the first embodiment are given the same signs and are not described.

FIG. 5 is a diagram showing the configuration of the distal direction side part of a treatment instrument 50 according to the second embodiment. FIG. 6 is a sectional view taken along line VI-VI of FIG. 5. As shown in FIG. 5, the treatment instrument 50 includes a treatment instrument body 24 and a distal treatment portion 26 similarly to the treatment instrument 20 in the first embodiment. A rotary joint portion 27 is provided between the treatment instrument body 24 and the distal treatment portion 26.

As shown in FIG. 5 and FIG. 6, in the outer peripheral surface of the distal direction side part of the treatment body 24 of the treatment instrument 50, projections 51 projecting toward the outer peripheral side are provided side by side in the circumferential direction of the treatment instrument 50. The projections 51 are provided in a state that the projections 51 have a predetermined length in the longitudinal direction. When the treatment instrument 50 is inserted through a treatment instrument insertion passage 18, each of the projections 51 is in contact with an inner peripheral surface 17. In such a configuration, when the treatment instrument body 24 is rotated around the axis relative to an endoscope 2, frictional force is applied in a direction opposite to the rotational direction of the treatment instrument body 24 at a contact between the projections 51 and the distal direction side part of the inner peripheral surface 17. That is, the projections 51 serve as a frictional force application portion which is configured to apply the frictional force to the contact portion between the distal direction side part of the inner peripheral surface 17 and the projections 51 (the outer peripheral surface in the distal direction side part of the treatment instrument body 24). The frictional force applied by the projections 51 regulates the rotation of the distal direction side part of the treatment instrument body 24 around the axis. The back-and-forth motion of the treatment instrument 50 relative to the endoscope 2 in the longitudinal direction is not regulated by the projections 51.

Now, the function of an endoscope device 1 according to the present embodiment is described. In the endoscope device 1, the treatment instrument 50 is inserted through the treatment instrument insertion passage 18 of the endoscope 2 to carry out a treatment. In the treatment using the endoscope device 1, a tissue or the like may be gripped by a gripping portion 28 of the distal treatment portion 26. During the gripping action performed by the gripping portion 28, force is applied to the gripping portion 28 provided in the distal direction side to the rotary joint portion 27. If the rotary joint portion 27 is actuated in this condition to rotate the distal treatment portion 26, the distal direction side part of the treatment instrument body 24 provided in the proximal direction side to the rotary joint portion 27 rotates in a direction opposite to the rotational direction of the rotational operation.

In the endoscope device 1, each of the projections 51 is in contact with the inner peripheral surface 17 when the treatment instrument 50 is inserted through the treatment instrument insertion passage 18. Therefore, when the treatment instrument body 24 is rotated around the axis relative to the endoscope 2, frictional force is applied in a direction opposite to the rotational direction of the treatment instrument body 24 at the contact portion between the distal direction side part of the inner peripheral surface 17 and the projections 51 (the outer peripheral surface in the distal direction side part of the treatment instrument body 24). The frictional force applied by the projections 51 regulates the rotation of the distal direction side part of the treatment instrument body 24 in a direction opposite to the rotational direction of the rotational operation. The around-the-axis rotation of the distal direction side part of the treatment instrument body 24 provided in the proximal direction side to the rotary joint portion 27 is regulated. As a result, even when the gripping portion 28 is gripping a tissue or the like (force is being applied to the distal treatment portion 26), the rotational operation is properly transmitted to the distal treatment portion 26 provided in the distal direction side to the rotary joint portion 27. Thus, the distal treatment portion 26 properly rotates around the axis relative to the treatment instrument body 24.

Therefore, the endoscope device 1 having the configuration described above has the following advantages. That is, in the endoscope device 1 according to the present embodiment, when the treatment instrument 50 is inserted through the treatment instrument insertion passage 18, each of the projections 51 is in contact with the inner peripheral surface 17. Therefore, when the treatment instrument body 24 is rotated around the axis relative to the endoscope 2, frictional force is applied in a direction opposite to the rotational direction of the treatment instrument body 24 at the contact portion between the distal direction side part of the inner peripheral surface 17 and the projections 51 (the outer peripheral surface in the distal direction side part of the treatment instrument body 24). The frictional force applied by the projections 51 regulates the rotation of the distal direction side part of the treatment instrument body 24 in a direction opposite to the rotational direction of the rotational operation when the distal treatment portion 26 is rotated in a state that the gripping portion 28 is gripping a tissue or the like (force is being applied to the distal treatment portion 26). The around-the-axis rotation of the distal direction side part of the treatment instrument body 24 provided in the proximal direction side to the rotary joint portion 27 is regulated. As a result, even when the force is being applied to the distal treatment portion 26, the rotational operation can be properly transmitted to the distal treatment portion 26 provided in the distal direction side to the rotary joint portion 27.

### (Modification of First and Second Embodiments)

The first and second embodiments may be modified as follows. As shown in FIG. 7, first projections 41 projecting toward the inner peripheral side are provided in the distal direction side part of an inner peripheral surface 17, and second projections 51 projecting toward the outer peripheral side are provided in the outer peripheral surface of the distal direction side part of a treatment instrument body 24 of a treatment instrument 55. In the endoscope device 1, the first projections 41 are in contact with the second projections 51 when the treatment instrument 55 is inserted through the treatment instrument insertion passage 18. In such a configuration, when the treatment instrument body 24 is rotated around the axis relative to an endoscope 2, frictional force is applied in a direction opposite to the rotational direction of the treatment instrument body 24 at the contact portion between the first projections 41 and the second projections 51. That is, the first projections 41 and the second projections 51 serve as a frictional force application portion which is configured to apply the frictional force to the contact portion between the first projections 41 (the distal direction side part of an inner peripheral surface 17) and the second projections 51 (the outer peripheral surface in the distal direction side part of the treatment instrument body 24).

As described above, according to the first and second embodiments and the modification thereof, the endoscope device 1 of the present invention may include the first projections 41 projecting toward the inner peripheral side from the distal direction side part of an inner peripheral surface 17. The endoscope device 1 may also includes the second projections 51 projecting toward the outer peripheral side from the outer peripheral surface in the distal direction side part of the treatment instrument body 24. In such a configuration, when the treatment instrument body 24 is rotated around the axis relative to an endoscope 2, the first projections 41 and/or the second projections 51 serve as a frictional force application portion which configured to apply the frictional force to the contact portion between the distal direction side part of the inner peripheral surface 17 and the outer peripheral surface in the distal direction side part of the treatment instrument body 24.

### (Third Embodiment)

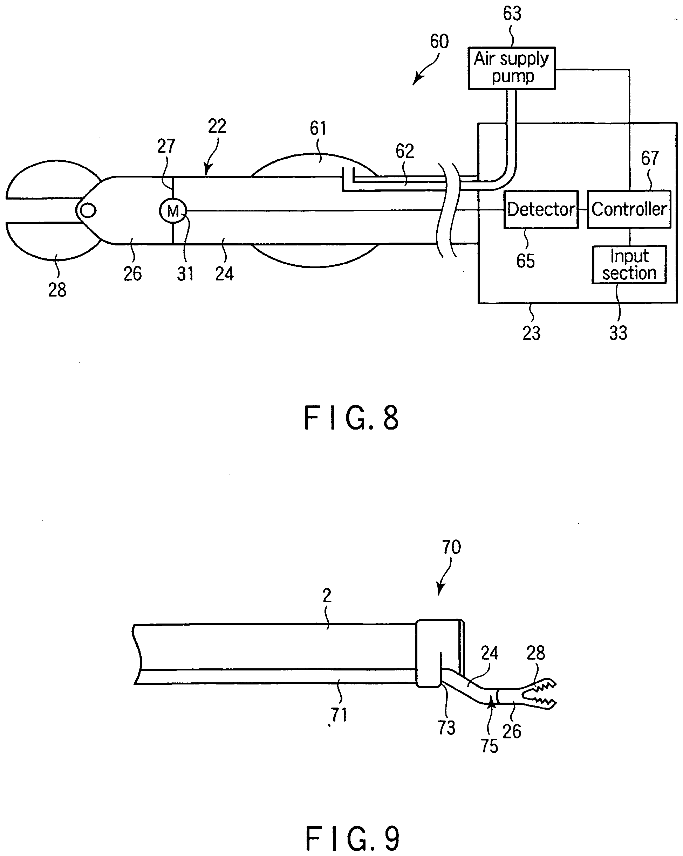

Now, a third embodiment is described with reference to FIG. 8. It should be noted that components having the same function as those in the first embodiment are given the same signs and are not described.

FIG. 8 is a diagram showing a treatment instrument 60 according to the third embodiment. As shown in FIG. 8, the treatment instrument 60 includes a treatment instrument insertion portion 22 and a treatment instrument operation portion 23 similarly to the treatment instrument 20 in the first embodiment. The treatment instrument insertion portion 22 includes a treatment instrument body 24 and a distal treatment portion 26. A rotary joint portion 27 is provided between the treatment instrument body 24 and the distal treatment portion 26.

In the outer peripheral surface in the distal part of the treatment instrument body 24, a balloon 61 is provided in a state that the balloon 61 has a predetermined length in the longitudinal direction. The distal end of an air supply tube 62 is connected to the balloon 61. The proximal end of the air supply tube 62 is connected to an air supply pump 63 provided outside the treatment instrument 60 through the treatment instrument body 24 and the treatment instrument operation portion 23. As in an endoscope device disclosed in Jpn. Pat. Appln. KOKAI Publication No. 2001-327460, the balloon 61 is inflated by supplying air to the balloon 61 from the air supply pump 63 via the air supply tube 62. As a result, the distal direction side part of the treatment instrument body 24 can be increased in diameter.

The treatment instrument operation portion 23 is provided with a detector 65 connected to a motor 31 of the rotary joint portion 27. The detector 65 detects the actuation of the rotary joint portion 27 (the rotation of the distal treatment portion 26 around the axis). The detector 65 is connected to a controller 67 provided in the treatment instrument operation portion 23. An input section 33 of the treatment instrument operation portion 23 and the air supply tube 62 are connected to the controller 67. In such a configuration, the balloon 61 can be manually inflated by the operation in the input section 33. When the actuation of the rotary joint portion 27 is detected by the detector 65, the air supply pump 63 may be controlled by the controller 67 so that air is supplied to the balloon 61. Thus, the balloon 61 can be automatically inflated in accordance with the actuation of the rotary joint portion 27.

When the treatment instrument 60 is inserted through a treatment instrument insertion passage 18 and when the balloon 61 is inflated, the balloon 61 is in contact with an inner peripheral surface 17. In such a configuration, when the treatment instrument body 24 is rotated around the axis relative to an endoscope 2, frictional force is applied in a direction opposite to the rotational direction of the treatment instrument body 24 at a contact portion between the balloon 61 and the distal direction side part of the inner peripheral surface 17. That is, the balloon 61 serves as a frictional force application portion which is configured to apply the frictional force to the contact portion between the distal direction side part of the inner peripheral surface 17 and the balloon 61 (the outer peripheral surface in the distal direction side part of the treatment instrument body 24). The frictional force applied by the balloon 61 regulates the rotation of the distal direction side part of the treatment instrument body 24 around the axis. The deflation of the balloon 61 enables the back-and-forth motion of the treatment instrument 60 relative to the endoscope 2 in the longitudinal direction.

Now, the function of an endoscope device 1 according to the present embodiment is described. In the endoscope device 1, the treatment instrument 60 is inserted through the treatment instrument insertion passage 18 of the endoscope 2 to carry out a treatment. In the treatment using the endoscope device 1, a tissue or the like may be gripped by a gripping portion 28 of the distal treatment portion 26. During the gripping action performed by the gripping portion 28, force is applied to the gripping portion 28 provided in the distal direction side to the rotary joint portion 27. If the rotary joint portion 27 is actuated in this condition to rotate the distal treatment portion 26, the distal direction side part of the treatment instrument body 24 provided in the proximal direction side to the rotary joint portion 27 rotates in a direction opposite to the rotational direction of the rotational operation. Moreover, when the rotary joint portion 27 is actuated, the actuation of the rotary joint portion 27 is detected by the detector 65, and the air supply pump 63 is controlled by the controller 67 so that air is supplied to the balloon 61. Thus, the balloon 61 is inflated, and the distal direction side part of the treatment instrument body 24 is increased in diameter. In this case, the balloon 61 may be manually inflated by the operation of the input section 33 of the treatment instrument operation portion 23.

In the endoscope device 1, when the treatment instrument 60 is inserted through the treatment instrument insertion passage 18 and when the balloon 61 is inflated, the balloon 61 is in contact with the inner peripheral surface 17. Therefore, when the treatment instrument body 24 is rotated around the axis relative to the endoscope 2, frictional force is applied in a direction opposite to the rotational direction of the treatment instrument body 24 at the contact portion between the distal direction side part of the inner peripheral surface 17 and the balloon 61 (the outer peripheral surface in the distal direction side part of the treatment instrument body 24). The frictional force applied by the balloon 61 regulates the rotation of the distal direction side part of the treatment instrument body 24 in a direction opposite to the rotational direction of the rotational operation. The around-the-axis rotation of the distal direction side part of the treatment instrument body 24 provided in the proximal direction side to the rotary joint portion 27 is regulated. As a result, even when the gripping portion 28 is gripping a tissue or the like (force is being applied to the distal treatment portion 26), the rotational operation can be properly transmitted to the distal treatment portion 26 provided in the distal direction side to the rotary joint portion 27. Thus, the distal treatment portion 26 properly rotates around the axis relative to the treatment instrument body 24.

Therefore, the endoscope device 1 having the configuration described above has the following advantages. That is, in the endoscope device 1 according to the present embodiment, when the treatment instrument 60 is inserted through the treatment instrument insertion passage 18 and when the balloon 61 is inflated, the balloon 61 is in contact with the inner peripheral surface 17. Therefore, when the treatment instrument body 24 is rotated around the axis relative to the endoscope 2, frictional force is applied in a direction opposite to the rotational direction of the treatment instrument body 24 at the contact portion between the distal direction side part of the inner peripheral surface 17 and the balloon 61 (the outer peripheral surface in the distal direction side part of the treatment instrument body 24). The frictional force applied by the balloon 61 regulates the rotation of the distal direction side part of the treatment instrument body 24 in a direction opposite to the rotational direction of the rotational operation when the distal treatment portion 26 is rotated in a state that the gripping portion 28 is gripping a tissue or the like (force is being applied to the distal treatment portion 26). The around-the-axis rotation of the distal direction side part of the treatment instrument body 24 provided in the proximal direction side to the rotary joint portion 27 is regulated. As a result, even when the force is being applied to the distal treatment portion 26, the rotational operation can be properly transmitted to the distal treatment portion 26 provided in the distal direction side to the rotary joint portion 27.

### (Other Modifications)

In the embodiments described above, the frictional force application portion includes the first projections 41 projecting toward the inner peripheral side from the distal direction side part of the inner peripheral surface 17 and the second projections 51 projecting toward the outer peripheral side from the outer peripheral surface in the distal direction side part of the treatment instrument body 24.

Furthermore, in the embodiments described above, the rotary joint portion 27 is provided with the motor 31, and the rotary joint portion 27 is actuated by driving the motor 31 so that the distal treatment portion 26 is rotated relative to the treatment instrument body 24. However, the configuration of actuating the rotary joint portion 27 is not limited to this configuration. For example, the rotary joint portion 27 may be provided with a bevel gear to which a rotational operation transmission member such as a wire is connected. In this treatment instrument, the back-and-forth motion of the rotational operation transmission member in the longitudinal direction drives the bevel gear, and the rotary joint portion 27 is actuated.

Still further, in the embodiments described above, the inner peripheral surface 17 that defines the treatment instrument insertion passage 18 to insert the treatment instrument is provided in the endoscope 2, the present invention is not limited to this. For example, as shown in FIG. 9, in an endoscope device 70, a tubular member 71 separate from the endoscope 2 is provided. In this case, the tubular member 71 may be provided with an inner peripheral surface 73 that defines a treatment instrument insertion passage (not shown) to insert a treatment instrument 75.

## Claims

1. An endoscope device (1, 70) comprising:
an endoscope (2);
a treatment instrument (20, 50, 55, 60, 75), the treatment instrument (20, 50, 55, 60, 75) including a treatment instrument body (24) extended in a longitudinal direction, and a distal treatment portion (26) provided in a distal direction side to the treatment instrument body (24); and
an inner peripheral surface (17, 73) which is provided in the endoscope (2) or in a tubular member (71) separate from the endoscope (2) and which defines a treatment instrument insertion passage (18) to insert the treatment instrument (20, 50, 55, 60, 75); **characterized in that**
the treatment instrument (20, 50, 55, 60, 75) comprises a rotary joint portion (27) which is provided between the treatment instrument body (24) and the distal treatment portion (26) and which is configured to rotate the distal treatment portion (26) around an axis relative to the treatment instrument body (24),
the endoscope device (1, 70) further comprises
a frictional force application portion (41, 51, 61) configured to apply frictional force to a contact portion between a distal direction side part of the inner peripheral surface (17, 73) and an outer peripheral surface in a distal direction side part of the treatment instrument body (24) when the distal treatment portion (26) is rotated around the axis relative to the treatment instrument body (24) in a state that a force is applied to the distal treatment portion (26), and thereby configured to regulate a rotation of the treatment instrument body (24) relative to the endoscope (2) towards a direction opposite to a rotational direction of the distal treatment portion, wherein the frictional force application portion (41, 51, 61) includes a projection (41) projecting, from the distal direction side part of the inner peripheral surface (17, 73), toward the outer peripheral surface in the distal direction side part of the treatment instrument body (24) such that the tip of the projection (41) is in contact with the outer peripheral surface, and
the frictional force application portion (41, 51, 61) includes first projections (41) as the projection (41) which project on the inner peripheral surface (17, 73) toward an inner peripheral side, and
second projections (51) which are provided side by side in a state that each of the second projections (51) has a predetermined length in the longitudinal direction, and with which the first projections (41) are in contact, the second projections (51) projecting on the outer peripheral surface of the treatment instrument body (24) toward an outer peripheral side.

2. The endoscope device (1, 70) according to claim 1, **characterized in that** the second projections (51) are located to a proximal direction side of the treatment instrument body (24) with respect to the rotary joint (27).

## Patentansprüche

1. Endoskopvorrichtung (1, 70) mit:
einem Endoskop (2);
einem Behandlungsinstrument (20, 50, 55, 60, 75), wobei das Behandlungsinstrument (20, 50, 55, 60, 75) einen Behandlungsinstrumentkörper (24), der sich in eine Längsrichtung erstreckt, und einen distalen Behandlungsteil (26), der auf einer Seite in distaler Richtung an dem Behandlungsinstrumentkörper (24) vorgesehen ist, aufweist; und
einer Innenumfangsfläche (17, 73), die in dem Endoskop (2) oder in einem von dem Endoskop (2) getrennten rohrförmigen Element (71) vorgesehen ist und die eine Behandlungsinstrument-Einführpassage (18) zum Einführen des Behandlungsinstruments (20, 50, 55, 60, 75) definiert; **dadurch gekennzeichnet, dass**
das Behandlungsinstrument (20, 50, 55, 60, 75) einen Drehgelenkteil (27) aufweist, der zwischen dem Behandlungsinstrumentkörper (24) und dem distalen Behandlungsteil (26) vorgesehen ist und der dazu ausgebildet ist, den distalen Behandlungsteil (26) um eine Achse relativ zu dem Behandlungsinstrumentkörper (24) zu drehen,
wobei die Endoskopvorrichtung (1, 70) ferner aufweist:
einen Reibungskraftaufbringungsteil (41, 51, 61), der dazu ausgebildet ist, Reibungskraft auf einen Kontaktteil zwischen einem Teil der Innenumfangsfläche (17, 73) auf der Seite in distaler Richtung und einer Außenumfangsfläche in einem Teil des Behandlungsinstrumentkörpers (24) auf der Seite in distaler Richtung aufzubringen, wenn der distale Behandlungsteil (26) um die Achse relativ zu dem Behandlungsinstrumentkörper (24) in einem Zustand gedreht wird, in dem eine Kraft auf den distalen Behandlungsteil (26) aufgebracht wird, und dadurch dazu ausgebildet ist, eine Drehung des Behandlungsinstrumentkörpers (24) relativ zu dem Endoskop (2) in eine Richtung zu regulieren, die einer Drehrichtung des distalen Behandlungsteils entgegengesetzt ist, wobei der Reibungskraftaufbringungsteil (41, 51, 61) einen Vorsprung (41) aufweist, der von dem Teil der Innenumfangsfläche (17, 73) auf der Seite in distaler Richtung zur Außenumfangsfläche in dem Teil des Behandlungsinstrumentkörpers (24) auf der Seite in distaler Richtung hervorragt, derart, dass die Spitze des Vorsprungs (41) in Kontakt mit der Außenumfangsfläche ist, und
wobei der Reibungskraftaufbringungsteil (41, 51, 61) aufweist
erste Vorsprünge (41) als den Vorsprung (41), der an der Innenumfangsfläche (17, 73) zu einer Innenumfangsseite hin hervorragt, und
zweite Vorsprünge (51), die Seite an Seite in einem Zustand vorgesehen sind, in dem jeder der zweiten Vorsprünge (51) eine vorbestimmte Länge in die Längsrichtung hat, und mit denen die ersten Vorsprünge (41) in Kontakt sind, wobei die zweiten Vorsprünge (51) auf der Außenumfangsseite des Behandlungsinstrumentkörpers (24) zu einer Außenumfangsseite hin hervorragen.

2. Endoskopvorrichtung (1, 70) nach Anspruch 1, **dadurch gekennzeichnet, dass** die zweiten Vorsprünge (51) sich an einer Seite des Behandlungsinstrumentkörpers (24) in proximaler Richtung bezüglich des Drehgelenks (27) befinden.

## Revendications

1. Dispositif (1, 70) d'endoscope comprenant :
un endoscope (2) ;
un instrument de traitement (20, 50, 55, 60, 75), l'instrument de traitement (20, 50, 55, 60, 75) comprenant un corps (24) d'instrument de traitement s'étendant dans une direction longitudinale, et une partie (26) de traitement distale prévue du côté de direction distale sur le corps (24) d'instrument de traitement ; et
une surface périphérique interne (17, 73) qui est prévue dans l'endoscope (2) ou dans un élément tubulaire (71) séparé de l'endoscope (2) et qui définit un passage d'insertion (18) d'instrument de traitement pour insérer l'instrument de traitement (20, 50, 55, 60, 75) ; **caractérisé en ce que**
l'instrument de traitement (20, 50, 55, 60, 75) comprend une partie de joint rotatif (27) qui est prévue entre le corps (24) d'instrument de traitement et la partie (26) de traitement distale et qui est configurée pour faire tourner la partie (26) de traitement distale autour d'un axe par rapport au corps (24) d'instrument de traitement,
le dispositif (1, 70) d'endoscope comprend en outre
une partie (41, 51, 61) d'application de force de frottement configurée pour appliquer une force de frottement sur une partie de contact entre une partie latérale de direction distale de la surface périphérique interne (17, 73) et une surface périphérique externe dans une partie latérale de direction distale du corps (24) d'instrument de traitement lorsque la partie (26) de traitement distale est mise en rotation autour de l'axe par rapport au corps (24) d'instrument de traitement dans un état dans lequel une force est appliquée à la partie (26) de traitement distale, et de ce fait configurée pour réguler une rotation du corps (24) d'instrument de traitement par rapport à l'endoscope (2) vers une direction opposée à la direction de rotation de la partie de traitement distale, dans lequel la partie (41, 51, 61) d'application de force de frottement comprend une saillie (41) faisant saillie, depuis la partie latérale de direction distale de la surface périphérique interne (17, 73), vers la surface périphérique externe dans la partie latérale de direction distale du corps (24) d'instrument de traitement d'une manière telle que la pointe de la saillie (41) est en contact avec la surface périphérique externe, et
la partie (41, 51, 61) d'application de force de frottement comprend
des premières saillies (41) en tant que saillie (41) se projetant sur la surface périphérique interne (17, 73) vers un côté périphérique interne, et
des deuxièmes saillies (51) qui sont prévues côte à côte dans un état dans lequel chacune des deuxièmes saillies (51) présente une longueur prédéterminée dans la direction longitudinale, et avec lesquelles les premières saillies (41) sont en contact, les deuxièmes saillies (51) se projetant sur la surface périphérique externe du corps (24) d'instrument de traitement vers un côté périphérique externe.

2. Dispositif (1, 70) d'endoscope selon la revendication 1, **caractérisé en ce que** les deuxièmes saillies (51) sont placées sur un côté de direction proximale du corps (24) d'instrument de traitement par rapport au joint rotatif (27).
